# EUROPEAN PATENT APPLICATION

(11) **EP 1 627 595 A1**
(43) Date of publication of application: **22.02.2006**
(21) Application number: 03706641.2
(22) Date of filing: 03.02.2003
(51) Int. Cl.: A61B 1/04

(54) **SYSTEM FOR THE ACTUAL VIEWING OF COLOURS ON SURFACES IN THE ABSENCE OF LIGHT, WHICH IS INTENDED FOR USE IN ENDOSCOPY**

(30) Priority: 08.07.2002 ES 200201419
(71) Applicant: Caballero Chaves, Manuel, E-11500 Cadiz (ES)
(72) Inventor: Caballero Chaves, Manuel, E-11500 Cadiz (ES)
(86) International application number: PCT/ES2003/000062
(87) International publication number: WO 2004/004552

(57) **Abstract**

Real colour vision system in dark surfaces endoscope that may use a xenon light source whose wavelength is never below 1100 nm with an intensity of light that is never higher than 0,000000001 lux or it may not use any light source. A signal image treatment circuit at the outlet of the camera amplifies the signal image and a monitor of a resolution of 1800 lines help to keep it in good working order. It has a parameter correction circuit, which permits the user improve the visualization in the monitor. A high frequency radio link avoids the need of a cable between the endoscope optic and the monitor.

## Description

### Object of the invention

The invention object is a real colour vision system in dark surfaces for endoscopy. If we try not to illuminate the area to examine, we will avoid undesirable shadows or reflections. On the other hand, we will be able to show the real colours, which appear in the surface; this would help us to achieve a subtler diagnostic.

### Precedents of the invention

### Endoscopy

An endoscopy allows physicians to peer through the body passageways through an endoscope. It carries the image of the body cavity back, supported by a luminous system. There is also a separate port that permits to wash, to suck up or to take samples.

There are two types of endoscopy procedures: one that uses an existing body opening to insert the tube, gastroscopy (mouth), colonoscopy (anus), etc. and other that requires a small puncture, laparoscopy (abdomen), thoracoscopy - (thorax), arthroscopy (joints), etc.

### Traditional endoscopes

It is a hermetic rigid tube enclosing an optic system made up of crystal lenses. The first way of illuminating is by an electric bulb, placed at the end of the endoscope. The problems would be the small size of the bulb, its fragility and the heat emission, which will make the tissue dry up. In the second way, the light comes from an external source that transmits an over-voltage and lets us take pictures or moving images.

### Fibroscopes

Fibroscopes light transmission is made by fiberglass put together in beams. The light conductor is constant from the light source to the end of the fibroscope in order to avoid a loss of light.

These fiberglasses must be put in the same order at the two ends of the beam so that the image can be transmitted correctly. The beams are called «coherent» and they transmit the image with the same amount of points as of beams.

The parts of a fibroscope are: a cold light generator (a 150W halogen headlamp or a short arched tube in xenon atmosphere), a cable which connects the generator and the machine, a control headboard where the ocular is and the main tube with its flexible end and its optic headboard.

### Video-endoscopes

An electronic one has replaced the optic system. A high-resolution miniaturized digital camera is placed at the end of the tube and it converts de luminous signal into electric impulses. A filter obtains three different pieces of information almost at the same time (one for each colour). We achieve the colour by coding and digitizing this information. This digital signal is transmitted to a central unit by a metal cable. The central unit, which has the same functions as a traditional endoscope and a computer image-editing program, synthesize the three pieces of information and converts them into moving images. These images are restored in a monitor.

Video-endoscopes let us do every endoscopy therapeutic operations (polypectomy, laser, haemostasis, catheterism, sphincterotomy...). The only restriction that we have nowadays is the size of the digital camera, which forces us to use electronic endoscopes that are minimum 9 mm in diameter (approximately).

These endoscopes have a better visibility and a sharper picture than a fibroscope, a high-resolution screen and an image record system.

### Laparoscopes

They are used to explore the abdomen in digestive, gynaecological, thoracic and renal surgery. A hollow tube is introduced through a small puncture; afterwards, the endoscope, generally rigid, is slipped into the tube.

These endoscopes may be used with different diameter, angle and length, depending on what part of the body we want to examine: hysteroscopes (through the cervical canal), urology (cistoscopies and resections), arthroscopy (joints), otolaryngology (otoscopy, sinuscopy, laryngoscopy, esophagoscopy and bronchoscopy) and other.

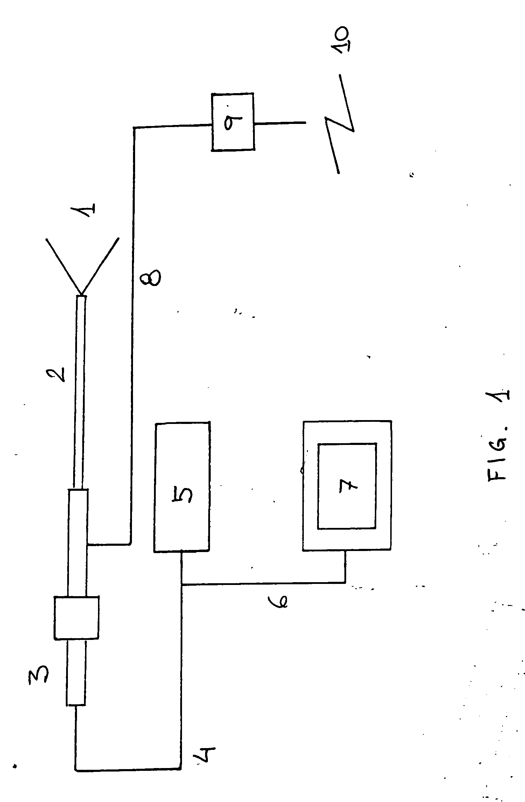

This kind of endoscope can be seen on FIG. 1, where 1 symbolizes the light beam that illuminates the surface.

They consist of:

### -Optic system.

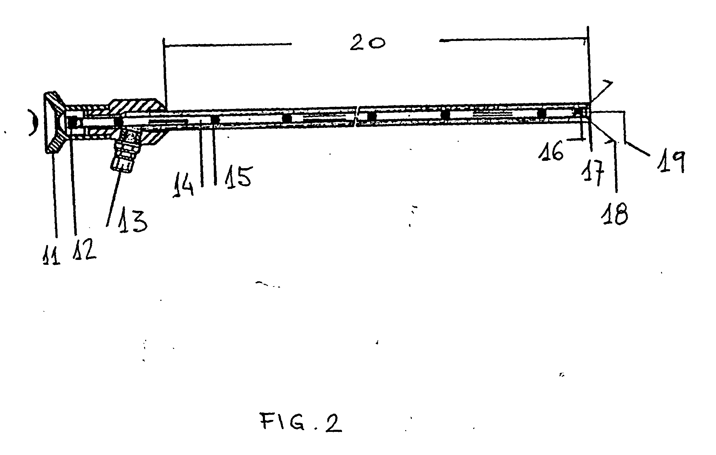

The optic system is made up of the laparoscopic optic (2), which can be seen on FIG. 2.

The working length (20) is cylindrical; its length is between 200 and 300 mm and its diameter 2 and 10 mm. It has an ocular (11), an ocular lens (12), chromatic lenses (15) and objective lens (16), a cable (8) connection (13) with optic fibers for the transmission of the light towards the exit of the light (17). The optics may have different fields of vision (18) and angles of vision (19), generally between 0 and 45 degrees.

### -Illumination system.

The laparoscopic optic (2) is illuminated by a light source (9) that transmits the light through an optic fiber cable (8). The light can be regulated and it works with an alternating current of 220V (10).

Many sorts of lamps may be used (halogen headlamp, metal halide lamps, xenon, etc.) in rising scale of temperature of colour. The temperature of colour has a better quality if it resembles the sunlight, so the quality will depend on how high the temperature is. We usually use high intensity xenon light. It normally has a 300W lamp, more luminous and appropriated for laparoscopic surgery than the halogen headlamps, which are less luminous and illuminate with a yellowish shade.

### -Electronic system.

The optic (2) is connected to the ocular (11) and this one to a digital camera (3) that transmits the image to a magnetoscope (5) and a monitor (7) by a wiring rigid connexion (4).

The cameras can be two or three-dimensional. The two-dimensional cameras are made up of a central unit, a headboard with an adaptation system to the optic ocular and a cable that connects to the monitor. The basic element of the digital camera is the CCD (Charge Couple Device). A CCD consists of a circuit board constituted of a matrix of photosensitive dots called pixels, a memory area that keeps the charges created by the pixels and an electric exit register.

There is another area called «memory area» protected from the light. This area, the CCD's sensitive surface and the electric exit register form the CCD chip.

The cameras usually have from one to three CCDs: the ones that have one CCD divide their pixels for the three primary colours (red, green and blue), whereas the ones that have three, use one for each primary colour. This is why the last ones take sharper pictures and the colours are more accurate.

This cameras resolution will depend on the number of horizontal lines and on the number of CCDs. For cameras with one CCD the resolution is between 470 and 560 lines of horizontal resolution, while for cameras with three CCDs, the resolution is of 750 lines approximately. The latest progresses in micro technology let us have cameras with three CCDs, as light as one with one CCD.

The cameras have focusing mechanisms to adjust the focal distance. They can be automatic or manual. Recently, we have seen available cameras that permit approach or move away the image. We can also find equipments where the optic and the electronic system are integrated in the same system.

Cameras that are able to make three-dimensional images are more complex. The stereoscopic vision, which creates a depth and relief sensation, makes easier the movements required in surgery. Two micro-cameras (put together in the same piece) and an optic with two trajectories of image conduction are employed to capture and transmit both images to the monitor. Because this monitor generates a double image, a pair of Shutter glasses is necessary. They make the left and right crystal be opened and closed alternatively. Also, the control system activates many mechanisms at the same time. An image projector, which receives the video signals generated by the monitor, is used for collective visualization. A shutter put in front of the projector allows the use of passive glasses, normally plastic or disposable ones.

The camera's ignition system and the electronic controls are normally out of the surgery field although some equipments already have those controls in the camera itself. The control balance of white tones is normally situated near the ignition system. Before doing the operation, we must set the balance of white tones, to determine in the camera the white standard according to the light source.

Another control we can find in the camera is the light intensity, which can be used in the light source, the camera or the central unit.

Once the image is captured, the user, by modifying the initial parameters, may correct the colorimetry, contrast, brightness and luminosity.

This image signal, corrected or not, may be given to the monitors, image digitization systems or image stock system.

The image digitization systems are digital treatment systems that improve the contrast and sharpness of laparoscopic images in real time. Because of this, we can see the structures more detailed. We obtain this contrast improvement by modifying the colour parameters of an image. To achieve this objective, we digitize the complete image and we process it digitally. The digitalis greys are undergone pixel-to-pixel and, depending on some criteria, a two-dimensional filtrate. Likewise, the whole image is processed before a matrix, so that we obtain a contrast and sharpness optic increase.

The image stock systems let us record the images in many ways (magnetoscope, video printer) and utilize different storage systems (diskettes, video or magnetic disks, CDs, high capacity hard disks, DVDs, etc).

There are also image combination systems that permit a second or third image system (gastroscope, radiologic system, ecography, exterior image).

### Video-laparoscope

It is a laparoscope with a zero degree vision angle and an integrated video chip. It includes a fixed focus, which guarantees a great depth of field without having to focus during the operation. The design of the light cable, signal cable and image documentation makes the job easier.

From what has been said, the following can be concluded: one of the most serious problems of the endoscopes is their need to use an external light source to be able to see and achieve subtle diagnostics and precise therapeutic endoscopic movements. This entails some problems:
1. The image seen by the doctors with the actual systems does not reflect the matter real colour. Throwing light on the matter alters its characteristics; this could cause a wrong diagnostic.
2. The light is thrown on the vision field: it occasions shadows or reflections, which is detrimental to the image.
3. The external light source is an independent machine and it needs space and a connection system.

### Description of the invention

There is no need to use an external light source with the camera we are presenting because it can see in absolute darkness the real colours. It shows the real image as it is not affected by any external illumination.

On the other hand, the system transmits the image signal via radio, so we avoid the use of cables between the endoscope and the monitor.

The system that we are presenting has many advantages:
1. The diagnostic rooms are simplified because we remove an unnecessary machine and an important amount of cables.
2. Our system takes the real image (sharpness, resolution and colour) without undesirable artefacts.
3. Our invention takes the real image so we will achieve a subtler diagnostic, as we can examine the possible changes of colour in the matter.

This new system is based on the fact that absolute darkness does not exist. The human eye can visualize spectrometry waves from 350 nm to 850 nm approximately. This means that if the human eye does not perceive illumination, it does not mean that radiation of the matter is gone in the whole spectrum. We consider that a physic phenomenon happens. This phenomenon has not been studied until now, because it has always been thought that the human eye needs light to perceive anything.

We obtain the image with its real colorimetry and characteristics, without modifications or external light.

### Brief description of the figures

Figure 1 shows a sketch of a current endoscope.
Figure 2 shows the composition of a conventional laparoscope optic system.
Figure 3 shows a sketch of an endoscope according to the invention.

### How to produce the invention

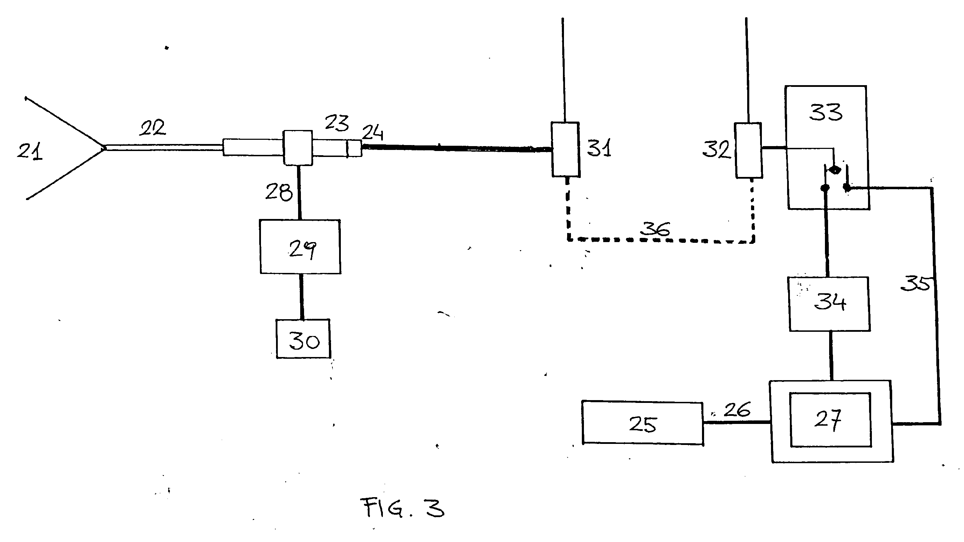

Figure 3 shows the system according to the invention. As figure 1, it shows an endoscopic system with three related systems: optic, illumination and electronic system.

Many modifications have been made in order to avoid the problems that exist in the present technology and include the real vision system. These modifications are:
- The optic (22) is connected to the camera (23) in a rigid way, to guarantee a solid and compact joint. It is a 10.5 or 2 mm diameter optic, with 0° or 30° of angle, depending on the surgeon's choice, with no light for the human eye.
- The electronic system is modified in many ways:
   - A circuit (24) is included in the outlet of the camera that enlarges the image signal.
   - The wiring rigid connection (4) between the camera and the monitor is replaced with a high frequency radio link that consists of a transceiver (31) and a recipient (32) supplemented with an emergency connection through an interface that is activated in case of a fault in the radio transmission.
   - A signal diverter (33) is included. It allows the operator chose the way of reproducing the images: directly (34) or through a correction circuit of image parameter (34) that permits the user correct the image parameter.
   - A colour monitor of 1800 lines (27) instead of a conventional one.

The illumination system uses a light source (29) of xenon at 1100 nm with a turn on switch, fed by 220v (30) and connected to the optic through an optic fiber cable (28). In this case, the existing entrance in the optic for the illumination is configured to work with or without illumination.

This system lets us use the current equipment and set of instruments:
- Veress needles
- Trocarts of 10/12, 10, 5 and 2 mm of diameter according to the surgery that will be done
- Hasson trocart
- Dissector cylinder trocart
- Reductants from 10 to 5, 5 to 2 m/m. diameter
- Apprehension forceps
- Endoscissors
- Endodissector
- Hook
- Laparoscopic needle carrier
- Clipper, clips, ligatures
- Laparoscopic mechanical suture
- Endobag
- Morcelator
- Wash and suck up system

The real colour vision system in dark surfaces is applicable in every use of the endoscopy, even if:
- they are flexible or rigid,
- they use an integral or distant camera,
- they are used for diagnosis or treatment,
- they have different thicknesses,
- they have an axial or lateral vision, regardless of the axis inclination degrees,
- the endoscope is used through an existing body opening or through a small puncture in any surgical or medical speciality.

On the other hand the real colour vision system in dark surfaces will be applicable to other medical uses, not only for endoscopic uses. These medical uses could employ photographic, video, or cinema cameras, with a view to observe and inspect (for example, the skin of a patient). It will also be applicable to biomechanical fields and to ergonomic studies, to an inspection of the bottom of the eye, or to observe the brain surgically.

We can also make use of the real colour vision system in dark surfaces in the industrial, scientific, security and defence fields, not only in endoscopes but in any photographic, video, or cinema cameras, any machine whose main objective is night vision, vision in the bottom of the sea or astronomy.

## Claims

1. Real colour vision system in dark surfaces uses a CCD digital camera and a television camera **characterized by** not using any light source for the illumination of the surface, and by including a signal image treatment circuit at the outlet of the camera. It amplifies the signal image taken by the camera and an 1800 lines monitor for the visualization of the taken image.

2. Real colour vision system in dark surfaces according to claim 1 **characterized by** including a circuit of manual correction of image parameters at the entrance of the monitor.

3. Real colour vision system in dark surfaces according to claim 2 **characterized by** including a signal diverter circuit. It is previous to the parameter correction circuit, which permits the user see in the monitor the signal image with or without parameter correction.

4. Real colour vision system in dark surfaces that uses a CCD digital camera and a television monitor **characterized** because the only light source the system utilizes is a lamp. It emits a radiation whose wavelength is equal or superior to 1100 nm with an intensity of light equal or lower to 0,000000001 lux. It includes a signal image treatment circuit at the outlet of the camera, which amplifies the signal image and the monitor has a horizontal resolution of 1800 lines.

5. Real colour vision system in dark surfaces according to claim 4 **characterized by** including a circuit of manual correction of image parameters at the entrance of the monitor.

6. Real colour vision system in dark surfaces according to claim 5 **characterized by** including a signal diverter circuit. It is previous to the parameter correction circuit, which permits the user see in the monitor the signal image with or without parameter correction.

7. Real colour vision system in dark surfaces endoscope which includes an optic system (22) which includes an ocular (11), an ocular lens (12), a series of lenses (15, 16) and connection means (13) to the light cable; an illumination system which includes means that generate light that may be adjustable (29) and an optic fiber cable (28) that throws light on the optic system. An electronic image system that includes image means of reception (23), image means of transmission, image means of visualization (27) and means to record images (25), **characterized by**:
- the connection means (13), placed in the optic (22), may open or close the light flow which comes from the light source (29),
- it does not use any light source for the surface illumination that is received by the image means of reception (23) and visualized by the image means of visualization (27),
- the connection between the ocular (11) and the image means of reception (23) is rigid,
- The image means of reception (23) include a CCD digital camera and a signal image treatment circuit at the outlet of the camera that amplifies the image signal with no illumination,
- The image means of visualization (27) include a monitor of 1800 lines for the visualization of the taken image without illumination.
- The signal generated by the image means of reception (23) is given to a high frequency radio transmitter (31) that sends it to a high frequency radio receiver (32) whose outlet is connected to the image means of visualization (27).
- It includes a synchronism circuit that synchronizes with the electromagnetic frequencies and deals in them for their later visualization in the image means of visualization (27).

8. Endoscope according to claim 7 **characterized by** the signal that the image means of visualization (27) receive. It goes through a circuit which corrects image parameters (35) and that allows the user adjust it.

9. Endoscope according to claim 8 **characterized by** the image signal which is taken by the image means of reception (23) that is given to a signal diverter circuit (33). It permits the user channel the signal correctly to the circuit which corrects image parameters (35) or directly (35) to the image means of visualization (27). This way the image parameters won't be corrected before their visualization so we will obtain the real colours of the explored surface.

10. Real colour vision system in dark surfaces endoscope which includes an optic system (22) which includes an ocular (11), an ocular lens (12), a series of lenses (15, 16) and connection means (13) to the light cable; an illumination system which includes means that generate light that may be adjustable (29) and a optic fiber cable (28) that throws light on the optic system. An electronic image system that includes image means of reception (23), image means of transmission, image means of visualization (27) and means to record images (25), **characterized by**:
- The means of illumination (29) illuminate the explored surface. It emits a radiation whose wavelength is never below 1100 nm with an intensity of light that is never higher than 0,000000001 lux during the system workings,
- the connection between the ocular (11) and the image means of reception (23) is rigid,
- the image means of reception (23) include a CCD digital camera and a signal image treatment circuit at the outlet of the camera, which amplifies the image signal with no illumination,
- the image means of visualization (27) include a monitor of 1800 lines for the visualization of the taken image without illumination.
- the signal generated by the image means of reception (23) is given to a high frequency radio transmitter (31) that sends it to a high frequency radio receiver (32) whose outlet is connected to the image means of visualization (27).
- It includes a synchronism circuit, which synchronizes with the electromagnetic frequencies and deals in them for their later visualization in the image means of visualization (27).

11. Endoscope according to claim 10 **characterized by** the signal that the image means of visualization (27) receive. It goes through a circuit which corrects image parameters (35) and that allows the user adjust it.

12. Endoscope according to claim 10 **characterized by** the image signal which is taken by the image means of reception (23) that is given to a signal diverter circuit (33). It permits the user channel the signal correctly to the circuit which corrects image parameters (35) or directly (35) to the image means of visualization (27). This way the image parameters will not be corrected before their visualization, so we will obtain the real colours of the explored surface.
